# EUROPEAN PATENT SPECIFICATION

(11) **EP 1 007 111 B1**
(45) Date of publication and mention of the grant of the patent: **14.09.2005**
(21) Application number: 96936132.8
(22) Date of filing: 02.10.1996
(51) Int. Cl.: A61M 1/00

(54) **FLUID-ASSISTED ELECTROCAUTERY DEVICE**
VORRICHTUNG ZUR ELEKTROKAUTERISATION UNTER MITWIRKUNG EINER GAS- ODER FLÜSSIGKEITSSTRÖMUNG
INSTRUMENT A FLUIDE POUR ELECTROCAUTERISATION

(43) Date of publication of application: 14.06.2000
(73) Proprietor: MEDTRONIC, INC., Minnesota 55432-5604 (US)
(72) Inventor: MULIER, Peter, M., J., Stillwater, MN 55082 (US); HOEY, Michael, F., Shorview, MN 55126 (US)
(74) Representative: Hughes, Andrea Michelle
(86) International application number: PCT/US1996/015796
(87) International publication number: WO 1998/014131

(56) References cited:
- WO-A-90/03152
- US-A- 1 735 271
- US-A- 4 326 529
- US-A- 5 167 659
- US-A- 5 401 272
- US-A- 5 472 441

## Description

This invention relates generally to the field of medical instruments, and more particularly relates to an electrocautery device.

Various types of electrocautery devices for incising and cauterizing body tissue are known and used in the medical field. Typically, such devices include a conductive blade or needle which serves as one electrode in an electrical circuit which is completed via a grounding electrode coupled to the patient. Incision of tissue is accomplished by applying a source of electrical energy (most commonly, a radio-frequency generator) to the blade. Upon application of the blade to the tissue, a voltage gradient is created, thereby inducing current flow and related heat generation at the point of contact. With sufficiently high levels of electrical energy, the heat generated is sufficient to cut the tissue and, advantageously to simultaneously cauterize severed blood vessels.

It is widely recognized in the prior art that the often substantial amount of smoke produced by electrocauterization of tissue is at least unpleasant, and in some cases distracting or even hazardous to the operator and other attending medical personnel. As a result, it has been proposed, and is common, to provide an electrocautery device with smoke-aspirating capabilities, such that the smoke produced from electrocauterization is quickly withdrawn from the area of incision. Smoke aspiration may be accomplished by providing, in the handle of the electrocautery device near the electrocautery blade/electrode, an inlet port to be coupled to a vacuum or suction source. Examples of this are described in U.S. Patent No. 4,307,720 to Weber, Jr., entitled "Electrocautery Apparatus and Method and Means for Cleaning the Same;" in U.S. Patent No. 5,242,442 to Hirschfeld, entitled "Smoke Aspirating Electrosurgical Device;" and in U.S. Patent No. 5.269.781 to Hewell, entitled "Suction Assisted Electrocautery Unit."

It has also been recognized in the prior art that the accumulation of coagulated blood, tissue rubble, and other debris on the electrode/blade of an electrocautery device can present a problem for the operator, necessitating the periodic cleaning of the blade, e.g., by wiping the blade over sterilized gauze or the like. This is generally regarded as undesirable, since the need to clean the electrode/blade tends to interrupt the incision procedure and increases the risks associated with contamination of the blade or the incision, damage to the blade, injury to the operator, and the like. To address this problem, it has been proposed in the prior art to provide an electrocautery instrument in which the electrode/blade is in slidable engagement with the instrument's handle, such that when the blade is retracted into the handle, any adhering debris is automatically scraped off onto the tip of the handle. Such an instrument is proposed in the above-referenced Weber, Jr. 720 patent. While this arrangement may have some benefit, it still may be necessary to wipe off the tip of the handle once the blade is retracted. It is believed that a more direct and effective approach to the problem would be to reduce the amount of debris created during the electrocautery process, thereby eliminating or at least reducing the need to clean the electrode/blade.

U.S. Patent No. 4,060,088 to Morrison Jr. et al., entitled "Electrosurgical Method and Apparatus for Establishing an Electrical Discharge in an Inert Gas Flow" describes a method and apparatus for coagulation by fulguration where the electrical discharge is established through a formation of flowing inert gas.

U.S. Patent No. 4,326,529 to Doss and Hutson, entitled "Corneal Shaping Electrode" describes a circulating saline electrode for charging corneal shape in eyes.

Document WO-A-90/03152 describes an electrosurgical apparatus having an inner suction tube and an outer fluid input tube.

In view of the foregoing considerations, the present invention is directed to an improved electrocautery instrument.

The invention provides a system for performing fluid-assisted electrocautery, said system comprising a fluid-assisted electrocautery instrument, comprising:
an elongate handle having proximal and distal ends and having a longitudinal lumen extending between said proximal and distal ends;
a conductive electrocautery electrode adapted to be coupled to a source of radio-frequency energy, said electrode comprising an elongate tube defining an internal lumen extending between proximal and distal ends of said electrode;
a fluid input tube, coupled to said proximal end of said electrode and in fluid communication with said internal lumen of said electrode, such that conductive fluid supplied from said input tube is communicated along said electrode and expelled from said distal end of said electrode; and
characterised by a suction tube, disposed partially within said lumen of said handle and having a distal end extending out of said distal end of said handle; wherein suction is applied to said suction tube during use so that air and fluid are drawn into the distal end of said suction tube; and wherein said electrode is disposed within said suction tube such that a distal end of said electrode extends distally beyond said distal end of suction tube; and said system further comprising a suction pump coupled to said proximal end of said suction tube.

In one embodiment of the invention, an electrocautery instrument is configured with an electrode/blade disposed within a retractable suction tube, such that with the suction tube advanced, the electrode/blade is concealed within the tube, and with the suction tube retracted, the distal end of the electrode/blade is exposed for performing electrocautery.

In accordance with one embodiment of the invention, the electrocautery electrode/blade is implemented with a hollow, conductive tube, flattened at its distal end into a blade-like configuration. Conductive fluid is applied to the proximal end of the hollow electrode/blade, and expelled from the distal (blade) end thereof during electrocautery. In accordance with another embodiment of the invention, the conductive fluid emanating from the electrode/blade conducts the RF electrocautery energy away from the blade, so that it is primarily the fluid, rather than the metal blade, which actually accomplishes the cutting of tissue. That is, the fluid serves as a "virtual" electrocautery electrode. Since it is the fluid, rather than the blade, which incises and cauterizes, no burns or perforations are made to the tissue, reducing the amount of debris in the incision. Also, the flow of fluid through the electrode/blade tends to keep the blade clean and cool.

The foregoing and other aspects of the present invention may perhaps be best appreciated with reference to a detailed description of a specific embodiment of the invention, given by way of example only, when read in conjunction with the accompanying drawings, wherein:
Figure 1 is a perspective view of an electrocautery instrument in accordance with one embodiment of the invention; and
Figure 2 is a enlarged perspective view of the distal end of the electrode/blade of the electrocautery instrument of Figure 1.

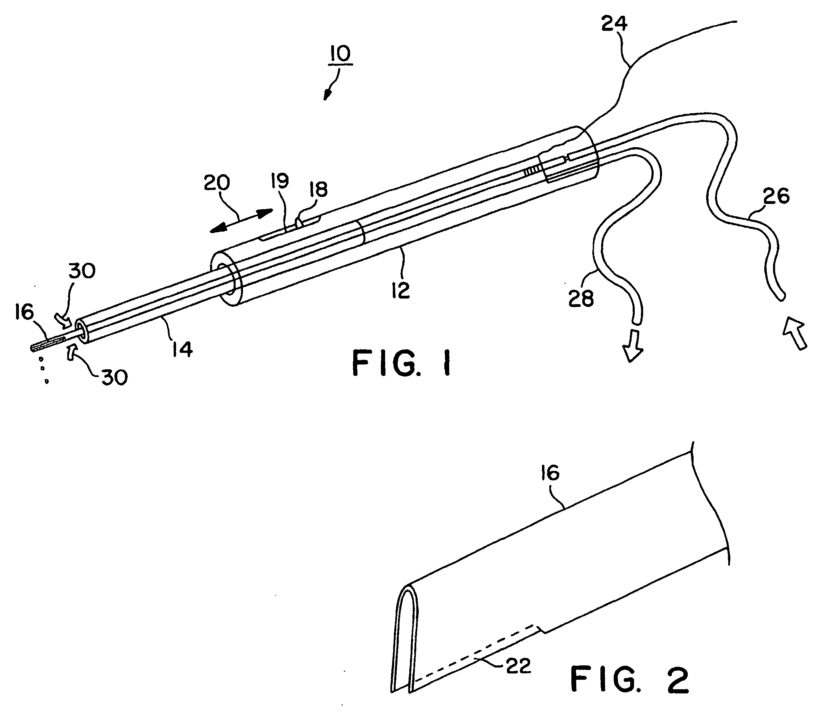

Referring to.Figure 1, there is shown a perspective view of a fluid-assisted electrocautery instrument 10 in accordance with one embodiment of the invention. Electrocautery instrument 10 comprises a handle 12, a suction tube 14, and an electrocautery electrode/blade 16. Handle 12 is preferably made of a sterilizable, rigid, and non-conductive material, such as nylon or the like. Suction tube 14, which is also preferably made of a sterilizable and non-conductive material, is slidably disposed partially within an internal lumen of handle 12, and projects distally out of the end thereof. Electrode/blade 16 is disposed within suction tube 14 and handle 12. Suction tube 14 is adapted to slide proximally and distally with respect to handle 12 and electrode 16 (i.e., in the directions of arrow 20 in Figure 1) by means of a sliding lever 18 extending out of a slot 19 in handle 12. With suction tube 14 in a retracted position, as shown in Figure 1, a distal portion of electrode/blade 16 projects beyond the distal end of tube 14, such that electrocautery can be performed. With suction tube in an advanced position, suction tube 14 completely conceals the tip of electrode/blade 16.

In accordance with one embodiment of the invention, electrode/blade 16 is preferably implemented using a hollow cylindrical tube which has been flattened at its distal end, as shown in the greatly enlarged perspective view of Figure 2. In addition to being flattened, a portion of the distal end of electrode/blade 16 is removed to form a longitudinal slit 22 therein.

Three connections are made to electrocautery instrument 10: One terminal (e.g., positive) of a radio-frequency (RF) generator (not shown in Figure 1) is electrically coupled to electrode/blade 16 via a wire 24; a source of fluid to be expelled from slit 22 in electrode/blade 16 is coupled to the proximal end of electrode/blade 16 via a flexible tube or hose 26; and a suction hose 28 is coupled to handle 12 so as to be in communication with the internal lumen of handle 12 and with suction tube 14. When suction is applied via hose 28, air and fluid are drawn into the distal end of suction tube 14, as indicated by arrows 30. The ability to advance or retract suction tube 14 with respect to electrode/blade 16 enables the operator of the instrument to perform electrocautery while simultaneously aspirating smoke and fluid from the incision site, or to use suction tube 14 alone, without performing electrocautery.

As noted above, conductive fluid is communicated from inflow tube 26 and communicated along the length of electrode/blade 16 to be expelled from the distal end thereof. This is done in order to establish a so-called virtual electrode for performing electrocautery. The infusion of conductive fluid simultaneously with the application of RF energy is discussed in further detail in: US patent number 5,431,649, in US patent number 5,609,151; and in US patent number 5,876,398. The foregoing patents are hereinafter collectively referred to as "the RF ablation applications".

As described in the RF ablation patents, the infusion of conducting fluid into the area of application of RF energy creates a "virtual electrode," the size and shape of which can be controllably modified, and which can be rendered more or less conductive, thereby modifying the spread of RF energy. By varying such factors as the RF energy and duration, the rate of infusion of conductive liquid, and the conductivity of the infused solution, the size, shape, and intensity of the "virtual electrode" -- i.e., the intensity of thermal production in the area, can be controlled. In the case of the electrocautery device in accordance with the present invention, application of the conductive solution during the application of RF energy further assists by preventing overheating of the electrode/blade, extending the point at which burning or charring of tissue would otherwise normally occur. To enhance this effect, it is contemplated that the solution being infused may first be cooled.

Conductive solutions believed to be suitable for establishing the virtual electrode include saline, saturated saline, and Ringer's solution, among others. Regarding the source of conductive fluid, it is contemplated that a conventional pump may be coupled to input line 26. Alternatively, it is contemplated that a small, pre-pressurized canister of conductive solution may be used, such that no pump is required. In one embodiment, handle 12 may be configured to receive such a pressurized canister therein, eliminating the need for input line 26.

Although in the embodiment of Figure 1, input line 26, suction line 28, and electrical connection 24 are depicted separately, it is contemplated that these connections to instrument 10 may be consolidated into a single line having two separate fluid-conducting lumens therein (one for input of conductive solution, one for suction), alongside an insulated electrical conductor.

Various alternative configurations of electrode/blade 16 are also contemplated. In one embodiment, a porous metal element is substituted for the flattened tube configuration of Figures 1 and 2.

From the foregoing detailed description of a specific embodiment of the invention, it should be apparent that a method and apparatus for performing fluid-assisted electrocautery of body tissue has been disclosed, wherein fluid delivered out of a hollow electrocautery electrode/blade creates a virtual electrode which incises and cauterizes the tissue.

## Claims

1. A system for performing fluid-assisted electrocautery, said system comprising a fluid-assisted electrocautery instrument (10), comprising:
an elongate handle (12) having proximal and distal ends and having a longitudinal lumen extending between said proximal and distal ends;
a conductive electrocautery electrode (16) adapted to be coupled to a source of radio-frequency energy, said electrode comprising an elongate tube defining an internal lumen extending between proximal and distal ends of said electrode;
a fluid input tube (26), coupled to said proximal end of said electrode and in fluid communication with said internal lumen of said electrode, such that conductive fluid supplied from said input tube is communicated along said electrode and expelled from said distal end of said electrode; a suction tube (14), disposed partially within said lumen of said handle and having a distal end extending out of said distal end of said handle; wherein suction is applied to said suction tube to draw air and fluid into the distal end of said suction tube (14); and wherein said electrode (16) is disposed within said suction tube (14) such that a distal end of said electrode extends distally beyond said distal end of suction tube; and said system further comprising
a suction pump coupled to said proximal end of said suction tube.

2. An electrocautery instrument in accordance with claim 1, wherein said distal end of said electrode (16) is flattened into a blade-like configuration.

3. An electrocautery instrument in accordance with claim 1 or 2, further comprising: a suction hose (28), adapted to be coupled between said proximal end of said suction tube and the suction pump, for aspirating smoke and fluid during electrocautery.

4. An electrocautery instrument in accordance with claim 1, 2 or 3 wherein said suction tube (14) is slidably disposed in said handle (12) such that said suction tube is slidable between a fully retracted position wherein a distal end of said electrode (16) extends beyond said distal end of said suction tube, and a fully advanced position wherein said distal end of said electrode is disposed within said suction tube.

## Patentansprüche

1. System zum Durchführen von fluidgestützter Elektrokauterisation bzw. Elektrokaustik, wobei das System ein fluidgestütztes Elektrokauterisationsinstrument (10) umfasst, mit:
einem langgestreckten Griff bzw. Stiel (12), der ein proximales und ein distales Ende und ein sich zwischen dem proximalen und dem distalen Ende erstreckendes longitudinales Lumen aufweist,
einer leitenden Elektrokauterisationselektrode (16), die dazu angepasst ist, an eine Hochfrequenzenergiequelle angeschlossen zu sein, wobei die Elektrode eine langgestreckte Röhre umfasst, die ein inneres Lumen definiert, das sich zwischen dem proximalen und dem distalen Ende der Elektrode erstreckt,
einer Fluideingaberöhre (26), die an das proximale Ende der Elektrode angeschlossen ist und in Fluidverbindung mit dem inneren Lumen der Elektrode steht, so dass von der Eingaberöhre geliefertes leitendes Fluid entlang der Elektrode übermittelt und von dem distalen Ende der Elektrode ausgestoßen wird,
einer Saugröhre (14), die teilweise innerhalb des Lumens des Griffs angeordnet ist und ein sich aus dem Distalende des Griffs erstreckendes Distalende aufweist, wobei ein Sog auf die Saugröhre beaufschlagt ist, um Luft und Fluid in das Distalende der Saugröhre (14) zu ziehen, und wobei die Elektrode (16) innerhalb der Saugröhre (14) angeordnet ist, so dass ein Distalende der Elektrode sich distal über das Distalende der Saugröhre hinaus erstreckt, und ferner mit
einer Saugpumpe, die mit dem proximalen Ende der Saugröhre in Verbindung steht.

2. Elektrokauterisationsinstrument nach Anspruch 1, bei dem das Distalende der Elektrode (16) zu einem klingenartigen Aufbau abgeflacht bzw. geglättet ist.

3. Elektrokauterisationsinstrument nach Anspruch 1 oder 2, ferner mit: einem Saugschlauch (28), der dazu angepasst ist, zwischen dem proximalen Ende der Saugröhre und der Saugpumpe angeschlossen zu sein, um Rauch und Fluid während Elektrokauterisation anzusaugen bzw. zu aspirieren.

4. Elektrokauterisationsinstrument nach Anspruch 1, 2 oder 3, bei dem die Saugröhre (14) gleitbar in dem Griff (12) angeordnet ist, so dass die Saugröhre zwischen einer vollständig zurückgezogenen Position, in der sich ein Distalende der Elektrode (16) über das Distalende der Saugröhre hinaus erstreckt, und einer vollständig vorgesetzten Position, in der das Distalende der Elektrode innerhalb der Saugröhre angeordnet ist, gleitbar ist.

## Revendications

1. Système pour effectuer une électrocautérisation assistée par un fluide, ledit système comportant un instrument d'électrocautérisation (10) assisté par un fluide, comportant :
une poignée allongée (12) ayant des extrémités proximale et distale, et ayant un lumen longitudinal s'étendant entre lesdites extrémités proximale et distale ;
une électrode d'électrocautérisation conductrice (16) adaptée pour être couplée à une source d'énergie radiofréquence, ladite électrode comportant un tube allongé définissant un lumen interne s'étendant entre les extrémités proximale et distale de ladite électrode ;
un tube d'entrée de fluide (26), couplé à ladite extrémité proximale de ladite électrode et en communication de fluide avec ledit lumen interne de ladite électrode, de telle sorte qu'un fluide conducteur alimenté à partir dudit tube d'entrée est mis en communication le long de ladite électrode et expulsé à partir de l'extrémité distale de ladite électrode ; un tube d'aspiration (14), disposé partiellement dans ledit lumen de ladite poignée, et ayant une extrémité distale s'étendant à l'extérieur de ladite extrémité distale de ladite poignée ; dans lequel une aspiration est appliquée audit tube d'aspiration pour attirer de l'air et du fluide dans l'extrémité distale dudit tube d'aspiration (14) ; et dans lequel ladite électrode (16) est disposée dans ledit tube d'aspiration (14) de telle sorte qu'une extrémité distale de ladite électrode s'étend de manière distale au-delà de ladite extrémité distale dudit tube d'aspiration ; et ledit système comportant en outre
une pompe d'aspiration couplée à ladite extrémité proximale dudit tube d'aspiration.

2. Instrument d'électrocautérisation selon la revendication 1, dans lequel ladite extrémité distale de ladite électrode (16) est aplatie en une configuration analogue à une lame.

3. Instrument d'électrocautérisation selon la revendication 1 ou 2, comportant de plus : un tuyau flexible d'aspiration (28), adapté pour être couplé entre ladite extrémité proximale dudit tube d'aspiration et la pompe d'aspiration, pour aspirer de la fumée et du fluide pendant une électrocautérisation.

4. Instrument d'électrocautérisation selon la revendication 1, 2 ou 3, dans lequel ledit tube d'aspiration (14) est disposé de manière coulissante dans ladite poignée (12) de telle sorte que ledit tube d'aspiration peut coulisser entre une position entièrement rétractée, dans laquelle une extrémité distale de ladite électrode (16) s'étend au-delà de ladite extrémité distale dudit tube d'aspiration, et une position entièrement avancée, dans laquelle ladite extrémité distale de ladite électrode est disposée dans ledit tube d'aspiration.
